# EUROPEAN PATENT APPLICATION

(11) **EP 4 009 646 A1**
(43) Date of publication of application: **08.06.2022**
(21) Application number: 21211768.3
(22) Date of filing: 01.12.2021
(51) Int. Cl.: H04N 21/43, A63B 24/00, G16H 20/30, H04N 21/4367, H04N 21/81, H04N 21/432, H04N 21/8547, A63B 71/06

(54) **METHOD AND SYSTEM FOR IMPROVING THE WORKOUT EXPERIENCE OF A USER**

(30) Priority: 04.12.2020 IT 202000029972
(71) Applicant: Technogym S.p.A., 47521 Cesena, Forli'-Cesena (IT)
(72) Inventor: GIUDICI, Fabrizio, 47521 CESENA, FORLI'-CESENA (IT); CASALI, Luca, 47521 CESENA, FORLI'-CESENA (IT)
(74) Representative: Mozzi, Matteo

(57) **Abstract**

A method (300) for enjoying an audio component of a multimedia content during a workout, comprising steps of:
- establishing (301), by a portable electronic device of the user, a data connection with a user interface of an exercise machine which may be employed by the user during a workout;
- providing (302) a video component of a multimedia content which may be used during the workout at the exercise machine which may be employed by the user for the workout;
- streaming (303) an audio component of the multimedia content to the portable electronic device of the user, adapted to perform the workout at the exercise machine;
- playing (304), by the portable electronic device of the user, the audio component of the multimedia content by means of an audio speaker operatively associated with the portable electronic device of the user during a playing time, by the user interface of the exercise machine, of the video component of the multimedia content by means of a display module of the user interface of the exercise machine.

## Description

### Field of the invention

The present invention relates to the field of fitness and, in particular, to a method and system for improving the workout experience of a user, in greater detail, to a method and system for enjoying an audio component of a multimedia content during a workout.

### Technological background of the invention

Nowadays, a user can use multimedia content during a workout on an exercise machine.

The multimedia content may be simple entertainment, for example a TV channel or a video on the Internet (YouTube), or preparatory to the workout, such as, for example, a film of a trainer who provides the user with voice messages to perform the workout according to an established workout program and other motivational messages and/or advice to help the user improve the workout performance.

The multimedia content has a video component which is used by the user by means of a display of the exercise machine, and an audio component which conventionally is used by the user by means of headsets connected in a wired or wireless manner directly to the exercise machine.

In this regard, such headsets are usually already those provided to the user such as the headsets of the mobile phone, tablet or multimedia reader (for example, the traditional MP3 audio file reader).

In order to connect the headsets to the exercise machine in a wireless manner (for example, by means of Bluetooth connection) or in a wired manner, there is a need for the user to follow a specific procedure so as to make the headsets actually operational.

Such a procedure might not be known by the user and/or not be very intuitive, therefore difficult to implement, especially when the user is not familiar with technology and/or for a user who is not very lucid because he/she is tired from a preceding workout performed shortly before on another exercise machine.

This could therefore result in the need to request for assistance or in any case, a waste of time also even if the operation is performed autonomously with the subsequent slowing of the workout preparation operations which, within a workout class, without the possibility of being able to immediately use the audio component of the video of the trainer, could compromise the correct outcome of the workout with respect to the other more timely components of the workout class.

Moreover, another drawback is associated with the fact that an established workout program could require the successive use of several exercise machines.

Therefore, each time the exercise machine is switched, the user would be required to perform a new connecting operation of the headsets to the exercise machine in order to use the audio component, with the inevitable interruption thereof switching from one exercise machine to the successive one, in addition to encountering the drawbacks mentioned above relative to the headset connection operation.

All this could cause further drawbacks in terms of time, in addition to possibly distracting the user from the actual performance of the workout.

Moreover, a further drawback is associated with the fact that the user has limited possibilities of personalizing the content which may be used during a workout on an exercise machine with these types of solutions.

Therefore, the experience perceived by the user during the workout as a whole appears discontinuous in the face of the difficulties which could be encountered, and this certainly goes against the increasingly felt need today to ensure for the user a workout experience which is as performing, satisfactory and pleasant as possible.

### Summary

It is the object of the present invention to devise and provide a method for improving the workout experience of a user, in particular a method for enjoying an audio component of a multimedia content during the workout, which allows the above drawbacks with reference to the known art to be at least partially solved, in particular which allows enjoying an audio component in the most reliable, simple and timely manner possible such as to ensure for the user an improved workout experience.

Such an object is achieved by a method according to claim 1.

Preferred embodiments of said method are defined in the dependent claims.

The object of the present invention is also a system for enjoying an audio component of a multimedia content during a workout.

### Brief description of the drawings

Further features and advantages of the method and system according to the invention will become apparent from the following description which illustrates preferred embodiments, given by way of indicative, non-limiting example, with reference to the accompanying drawings, in which:
- Figures 1a, 1b, 1c, 1d and 1e illustrate examples of an exercise machine which may be employed by a user to perform a physical activity;
- Figure 2 illustrates, by means of a block diagram, a system for enjoying an audio content during a workout according to an embodiment of the present invention, and
- Figure 3 shows, by means of a block diagram, a method for enjoying an audio content during a workout according to an embodiment of the present invention.

It is worth noting that equivalent or similar elements are indicated by the same numerical and/or alphanumerical reference in the aforesaid drawings.

### Detailed description

With reference to the aforesaid drawings, it is now described a system 200 for enjoying an audio component of a multimedia content during a workout, later also system for enjoying or simply system.

The multimedia content, described in greater detail later, is indicated in the drawings by the alphanumeric reference CM1.

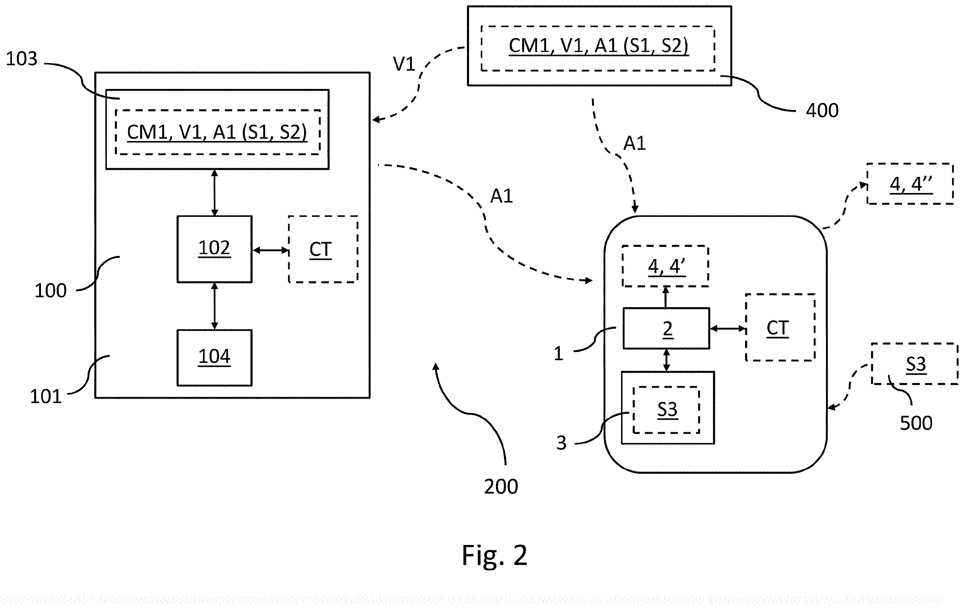

With reference to Figure 2, the system 200 comprises an exercise machine 100.

The exercise machine 100 may be any exercise machine which may be employed by a user to perform physical activity remotely (for example, from home), either alone or in a workout class, or to perform physical activity in a gym, either alone or in a workout class.

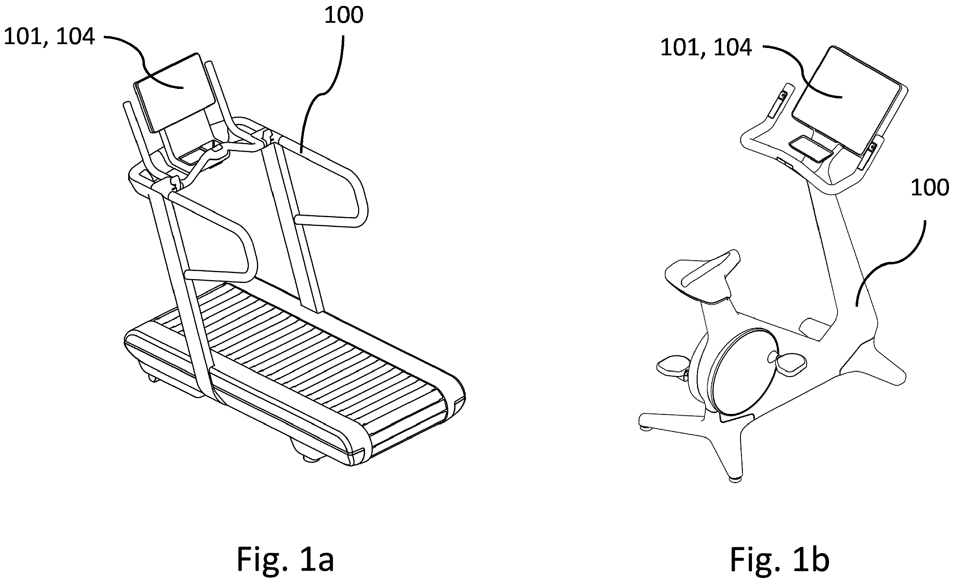

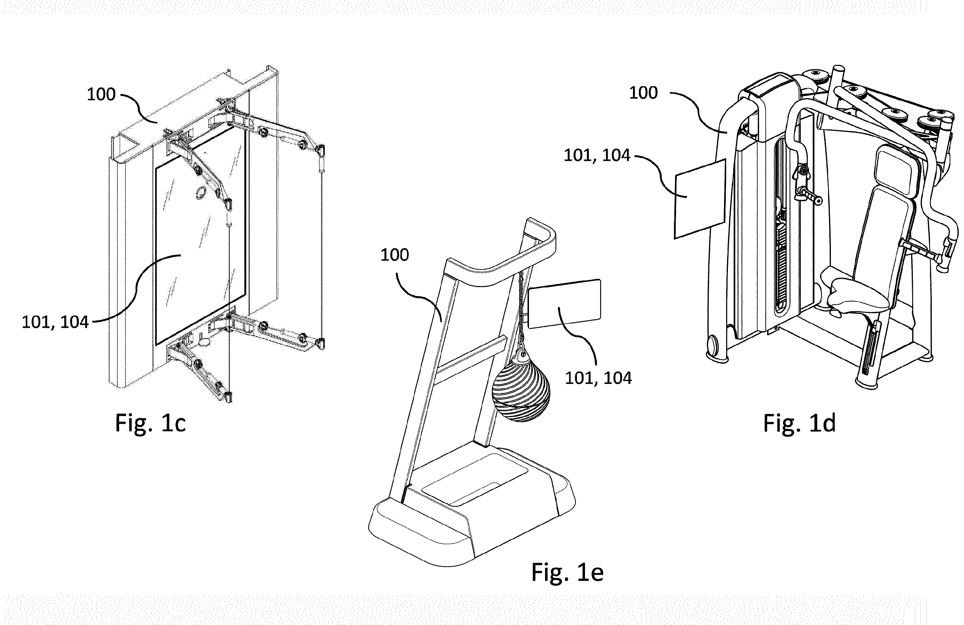

Examples of an exercise machine 100 are a treadmill (Figure 1a), a bike or an exercise bike (Figure 1b), a strength exercise machine (Figures 1c and 1d) and boxing equipment (Figure 1e), a rowing machine, an indoor cycling machine, and so on.

The following description - with reference in particular to Figure 2 - is valid for any exercise machine 100 listed above.

The exercise machine 100 comprises a user interface 101.

The user interface 101 comprises a data processing unit 102, for example a microprocessor or a microcontroller.

The user interface 101 further comprises a memory unit 103, operatively connected to the respective data processing unit 102.

The local memory unit 103 can be either internal or external (for example, as shown in Figure 2) to the data processing unit 102.

It is worth noting that the local memory unit 103 is configured to store one or more program codes which can be executed by the data processing unit 102 and data generated and processed following the execution of said one or more program codes.

In greater detail, as noted later, the memory unit 103 is configured to store the multimedia content CM1 which may be used by a user during the workout.

The data processing unit 102 of the user interface 101 is configured to control the operation of the exercise machine 100.

Moreover, the data processing unit 102 of the user interface 101 is configured to perform steps of the method for enjoying an audio component of a multimedia content during the workout, according to the present invention, as described below.

Returning in general to the user interface 101, it is configured to allow a user to interact with the exercise machine 100.

In particular, the user interface 101 is configured to allow the user to use the multimedia contents when performing the workout.

The multimedia content may be for entertainment purposes only (for example, Internet browsing, entertainment videos, audio/video music files, and so on), or for workout purposes such as, for example, audio messages and/or video clips which explain to the user how to use the exercise machine and/or perform the workout on the exercise machine according to a set workout program.

For the purposes of the present invention, reference will be made to the multimedia content such as a video of a trainer preparatory to the performance of a set workout program on the exercise machine, which video component, as described later, may be used by a user through the user interface 103 of the exercise machine 100.

In this regard, the multimedia content can be an audio and/or video of a trainer who provides the user with voice and/or gestural messages to perform the workout according to a set workout program (for example, how to change the setting of the exercise machine) and other motivational messages and/or useful tips that help the user to perform the workout correctly and improve his/her performance.

As diagrammatically shown in Figure 2, the multimedia content CM1 (audio and/or video of the trainer) comprises an audio component A1.

According to a further embodiment, in combination with the preceding one, the multimedia content CM1 preferably comprises a video component V1, in combination with the audio component A1.

With reference again to Figure 2, the user interface 101 of the exercise machine 100 further comprises a display module 104, for example, a display.

The display module 104 is operatively connected to the data processing unit 102 of the user interface 101.

With reference again to Figure 2, the system 200 further comprises a portable electronic device 1 of the user, for example, a mobile phone, a smartphone, a tablet, and so on.

The portable electronic device 1 comprises a respective data processing module 2, for example a microcontroller or microprocessor.

The portable electronic device 1 further comprises a respective memory module 3 operatively connected to the respective data processing module 2.

The memory module 3 may be internal or external (such as shown in Figure 2, for example) to the data processing module 2.

It is worth noting that the memory module 3 is configured to store one or more program codes which can be executed by the data processing module 2 and data generated and processed following the execution of said one or more program codes.

In addition to being configured to control the operation of the portable electronic device 1, the data processing module 2 is configured to perform steps of the method for enjoying an audio component of a multimedia content during the workout, according to the present invention, as described below.

With reference again to Figure 2, the system 200 further comprises an audio speaker 4 operatively associated with the portable electronic device 1 of the user, described later according to various embodiments of the invention.

In an embodiment, shown in Figure 2, the audio speaker 4 operatively associated with the portable electronic device comprises a loudspeaker 4' of the portable electronic device 1 of the user, for example a speaker.

According to a further embodiment, shown in Figure 2 and alternatively to or in combination with the preceding one, the audio speaker 4 operatively associated with the portable electronic device 1 of the user comprises a headset 4" configured to be operatively connected to the portable electronic device 1 of the user in a wireless or wired manner.

Returning in general to the system 200, according to an embodiment of the invention, the exercise machine 100 (the data processing unit 102 of the user interface 103, in particular) is configured to receive a video component V1 of a multimedia content CM1 which may be used during the workout.

In this embodiment, the user interface 101 (the respective data processing unit 102, in particular) is configured to play the video component V1 of the multimedia content CM1 by means of the display module 104 of the user interface 101 of the exercise machine 100.

In this embodiment, the portable electronic device 1 of the user (the respective data processing module 2, in particular) is configured to establish a data connection with the user interface 101 of the exercise machine 100 which may be employed by the user during a workout.

In this regard, both the portable electronic device 1 and the user interface 103 of the exercise machine 100 are configured to establish a data connection in wireless technology, for example in Bluetooth, Wi-Fi technology or similar.

It is worth noting that the data connection in wireless technology between the portable electronic device 1 of the user and the user interface 101 of the exercise machine 100 may be preferably established when the user is authenticated on the exercise machine 100.

For example, this may automatically occur via Bluetooth and/or by means of reading a code (a QR type code, for example) displayed on the exercise machine 100 and captured or photographed by the user by means of the portable electronic device 1.

Moreover, the portable electronic device 1 of the user (the respective data processing module 2, in particular) is configured to receive the streamed audio component A1, as described below according to various embodiments, of the multimedia content CM1.

The portable electronic device 1 of the user (the respective data processing module 2, in particular) is further configured to play, by means of an audio speaker 4 operatively associated with the portable electronic device 1 of the user, the audio component A1 of the multimedia content CM1 during the playing time of the video component V1 of the multimedia content by the display module 104 of the user interface 101 of the exercise machine 100.

Therefore, the user is capable of playing the audio component A1 of the multimedia content CM1 on his/her own portable electronic device 1, thus avoiding the connection thereof to the user interface 101 of the exercise machine 100.

In an embodiment, in combination with the preceding one, the user interface 101 (therefore, the respective data processing unit 102) of the exercise machine 100 and the portable electronic device 1 (therefore, the respective data processing module 2) of the user are configured to synchronize the video component V1 of the multimedia content CM1 and the audio component A1 of the multimedia content CM1 with each other during the respective playing time.

Thereby, possible delays or early playing of the audio component A1 with respect to the video component V1 of the multimedia content CM1 (and vice versa), which could make the comprehension of the multimedia content CM1 by the user difficult, can therefore be reduced as much as possible, or even eliminated.

In an embodiment, in combination with the preceding one, the user interface 101 (therefore, the respective data processing unit 102) of the exercise machine 100 and the portable electronic device 1 (therefore, the respective data processing module 2) of the user are configured to synchronize, during the respective playing time, the video component V1 of the multimedia content CM1 and the audio component A1 of the multimedia content CM1 by comparing the timestamps distributed along a playing time axis of the video component V1 of the multimedia content CM1 and timestamps distributed along a playing time axis of the audio component A1 of the multimedia content CM1.

Moreover, if the user momentarily pauses the playing time of the video component V1 of the multimedia content CM1, for example by means of the user interface 101 of the exercise machine 100, the portable electronic device 1 of the user is configured to interrupt the playing time of the audio component A1 of the multimedia content CM1 up to a successive resumption of the playing time of the video component V1 of the multimedia content CM1.

Vice versa, if the user momentarily pauses the playing time of the audio component A1 of the multimedia content CM1, for example by means of the portable electronic device 1 of the user, the user interface 101 of the exercise machine 100 is configured to interrupt the playing time of the video component V1 of the multimedia content CM1 up to a successive resumption of the playing time of the audio component A1 of the multimedia content CM1.

In an embodiment, shown in Figure 2, in combination with any one of those described above, the system 200 further comprises a remote electronic calculator 400, for example a cloud server, operatively connected to the user interface 101 of the exercise machine 100.

The connection between the remote electronic calculator 400 and the user interface 101 of the exercise machine 100 is a wired or wireless connection, by means of a data communication network (not shown in the drawings), for example Internet.

In this embodiment, in order to provide the video component V1 of the multimedia content CM1, the user interface 101 of the exercise machine 100, therefore the data processing unit 102, is configured to:
- receive the video component V1 of the multimedia content CM1 from the remote electronic calculator 400 operatively connected to the user interface 101 of the exercise machine 100;
- play the video component V1 of the multimedia content CM1 received from the remote electronic calculator 400 operatively connected to user interface 101 of the exercise machine 100.

In an embodiment, in combination with the preceding one, the remote electronic calculator 400 operatively connected to the user interface 101 of the exercise machine 100 is configured to stream the audio component A1 of the multimedia content CM1.

According to an embodiment, in combination with any one of those described above, the above-described memory unit 103 of the user interface 101 of the exercise machine 100 is configured to store the multimedia content CM1.

In this embodiment, the user interface 101 of the exercise machine 100 (therefore, the respective data processing unit 102) is configured to play the video component V1 of the multimedia content CM1 by playing the video component V1 of the multimedia content CM1 stored in the memory unit 103 of the user interface 101 of the exercise machine 100.

Therefore, in this embodiment, the video component V1 of the multimedia content CM1 is not provided by the remote electronic calculator 400 of the user interface 101, as in the embodiment described above, but it is already stored in the memory unit 103 of the user interface 101.

In an embodiment, in combination with the preceding one, the user interface 101 of the exercise machine 100 (therefore, the respective data processing unit 102), is configured to stream the audio component A1 of the multimedia content CM1 stored in the memory unit 103 of the user interface 101 of the exercise machine 100.

Therefore, in this embodiment, the portable electronic device 1 of the user receives the streamed audio component A1 of the multimedia content CM1 from the user interface 101 of the exercise machine 100.

According to an embodiment, alternatively to the preceding ones, the system 200 comprises a remote electronic calculator 400 operatively connected to the user interface 101 of the exercise machine 100.

In this embodiment, the user interface 101 of the exercise machine 100, therefore the data processing unit 102, can be configured to:
- receive the video component V1 of the multimedia content CM1 from the remote electronic calculator 400 operatively connected to the user interface 101 of the exercise machine 100;
- play the video component V1 of the multimedia content CM1 received from the remote electronic calculator 400 operatively connected to the user interface 101 of the exercise machine 100;
- stream the audio component A1 of the multimedia content CM1.

In this embodiment, the audio component A1 of the multimedia content CM1 is stored in the memory unit 103 of the user interface 101 of the exercise machine 100 because the multimedia content CM1 was previously downloaded from the remote electronic calculator 400 (cloud).

According to a further embodiment, in combination with any one of those described above, as already mentioned above, the multimedia content CM1 is a video of a trainer preparatory to the performance of the workout.

In an embodiment, shown in Figure 2, the audio component A1 of the multimedia content CM1 comprises at least one first audio sub-component S1 representative of the voice of the trainer.

In an embodiment, in combination with the preceding one, the portable electronic device 1 of the user, therefore the respective data processing module 2, is configured to allow the user to select, as background music to be played together with the at least one first audio sub-component S1 representative of the voice of the trainer, a further audio component S3 representative of a background music available in the portable electronic device 1 of the user.

In an embodiment, alternatively to or in combination with the preceding one, the user interface 101 of the exercise machine 100 (therefore the respective data processing unit 102) is configured to allow the user to select, as background music to be played together with the at least one first audio sub-component S1 representative of the voice of the trainer, the further audio component S3 representative of a background music available in the portable electronic device 1 of the user.

According to a further embodiment, in combination with any one of the preceding ones, the audio component A1 of the multimedia content CM1 further comprises at least one second audio sub-component S2 representative of a background music.

The at least one second audio component S2 is selected, for example from an audio streaming platform.

In an embodiment, in combination with the preceding one, the portable electronic device 1 of the user, therefore the respective data processing module 2, is configured to allow the user to select, as background music to be played together with the at least one first audio sub-component S1 representative of the voice of the trainer, one of the at least one second audio sub-component S2 representative of a background music of the audio sub-component A1 of the multimedia content CM1 and a further audio component S3 representative of a background music available in the portable electronic device 1 of the user.

In an embodiment, alternatively to or in combination with the preceding one, the user interface 101 of the exercise machine 100 (therefore the respective data processing unit 102) is configured to allow the user to select, as background music to be played together with the at least one first audio sub-component S1 representative of the voice of the trainer, one of the at least one second audio sub-component S2 representative of a background music of the audio component A1 of the multimedia content CM1 and the further audio component S3 representative of a background music available in the portable electronic device 1 of the user.

In an embodiment, shown with dashed lines in Figure 2, in combination with any one of those described above, the further audio component S3 representative of a background music available in the portable electronic device 1 of the user is stored in the memory module 3 of the portable electronic device 1 of the user.

In an embodiment, alternatively to the preceding one, shown with dashed lines in Figure 2, the further audio component S3 representative of a background music available in the portable electronic device 1 of the user is an audio which may be streamed on the portable electronic device 1 of the user (for example, Spotify).

The audio which may be streamed preferably is provided by the user to the portable electronic device 1 from a further remote electronic calculator 500 (diagrammatically shown in Figure 2), for example an audio streaming platform.

Therefore, in this embodiment, the user has the possibility of listening to the voice of the trainer with a background music selected by the same user from the one already present in the audio component A1 of the multimedia content CM1 (second sub-component S1) and a preferred one (further audio component S3) already stored beforehand in the portable electronic device 1 of the user or which may be streamed from a further remote electronic calculator 500.

In a further embodiment, in combination with any one of those described above, the audio speaker 4 operatively associated with the portable electronic device comprises a loudspeaker 4' of the portable electronic device 1 of the user.

In this embodiment, the portable electronic device 1, therefore the respective data processing module 2, of the user is configured to play the audio component A1 of the multimedia content CM1 by means of the loudspeaker 4' of the portable electronic device 1 of the user during the playing time of the video component V1 of the multimedia content CM1 by the user interface 101 of the exercise machine 100 by means of the display module 104.

In a further embodiment, alternatively to or in combination with the preceding one, the audio speaker 4 operatively associated with the portable electronic device 1 of the user comprises a headset 4" configured to be operatively connected to the portable electronic device 1 of the user in a wireless (for example, by means of Bluetooth, Wi-Fi connection technology and so on) or wired (for example, by means of cable with jack coupling) manner.

In this embodiment, the portable electronic device 1 of the user is configured to play the audio component A1 of the multimedia content by means of the headset 4" operatively connected to the portable electronic device 1 of the user in a wireless or wired manner during the playing time of the video component V1 of the multimedia content CM1 by the user interface 101 of the exercise machine 100 by means of the display module 104.

According to a further embodiment, in combination with any one of those described above and shown with dashed lines in Figure 2, the user interface 101 comprises an activation command CT.

In this embodiment, the portable electronic device 1, therefore the respective data processing module 2, of the user is configured to receive and recognize the activation command CT provided by the user by means of the user interface 101 of the exercise machine 100.

An example of activation command CT may be a start session command or a start workout class with the trainer command, following the selection by the user, by means of the user interface 101 of the exercise machine 100.

The user interface 101 of the exercise machine 100 and the portable electronic device 1 of the user adapted to perform the workout at the exercise machine 100 are configured to receive the video component V1 of the multimedia content CM1 which may be used during the workout and receive the streamed audio component A1 of the multimedia content CM1, respectively, following the establishment, by the portable electronic device 1 of the user, of the data connection with the user interface 101 of the exercise machine 100, which may be employed by the user during a workout, in response to an activation command CT provided by the user by means of the user interface 101 of the exercise machine 100 and received and recognized by the portable electronic device 1 of the user.

According to a further embodiment, alternatively to or in combination with the preceding one and shown with dashed lines in Figure 2, the portable electronic device 1 of the user comprises an activation command CT.

In this embodiment, the user interface 101 of the exercise machine 100, therefore the respective portable electronic device 102, is configured to receive and recognize the activation command CT provided by the user by means of the data processing unit 1.

An example of activation command CT may be a start session command or a start workout class with the trainer command, following the selection by the user, by means of the portable electronic device 1 of the user.

The user interface 101 of the exercise machine 100 and the portable electronic device 1 of the user adapted to perform the workout at the exercise machine 100 are configured to receive the video component V1 of the multimedia content CM1 which may be used during the workout and receive the streamed audio component A1 of the multimedia content CM1, respectively, following the establishment, by the portable electronic device 1 of the user, of the data connection with the user interface 101 of the exercise machine 100, which may be employed by the user during a workout, in response to the activation command CT provided by the user by means of the portable electronic device 1 of the user.

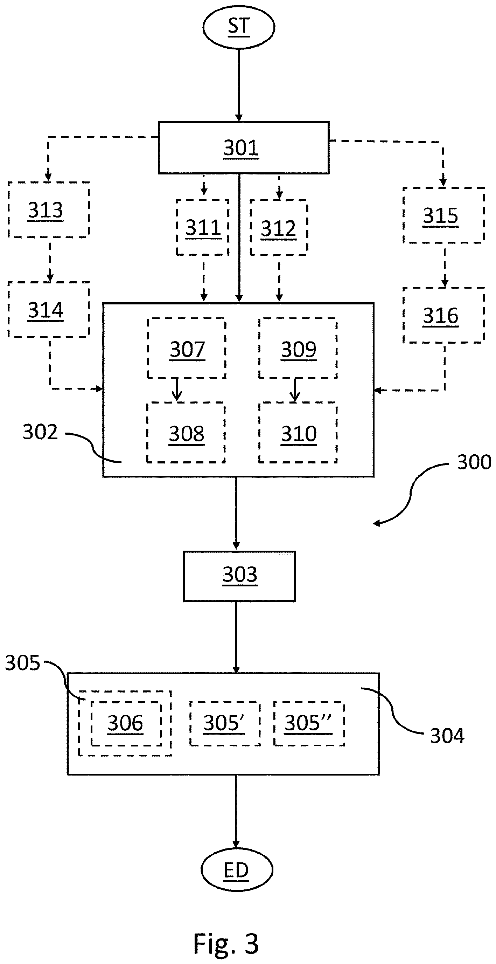

With reference now also to Figure 3, a method 300 for enjoying an audio component of a multimedia content during a workout, later also method for enjoying or simply method, is now described.

The method 300 comprises a symbolic step of starting ST.

The method 300 comprises a step of establishing 301, by a portable electronic device 1 of the user, a data connection with a user interface 101 of an exercise machine 100 which may be employed by the user during a workout.

The portable electronic device 1 of the user and the user interface 101 of the exercise machine 100 were already described above.

The method 300 further comprises a step of providing 302 a video component V1 of a multimedia content CM1 which may be used during the workout at the exercise machine 100, which may be employed by the user for the workout.

The method 300 further comprises a step of streaming 303 an audio component A1 of the multimedia content CM1 to the portable electronic device 1 of the user adapted to perform the workout at the exercise machine 100.

The multimedia content CM1 was already described above.

The method 300 further comprises a step of playing 304, by the portable electronic device 1 of the user, the audio component A1 of the multimedia content CM1 by means of an audio speaker 4 operatively associated with the electronic device 1 of the user during a playing time, by the user interface 101 of the exercise machine 100, of the video component V1 of the multimedia content CM1 by means of a display module 104 of the user interface 101 of the exercise machine 100.

In an embodiment, shown with dashed lines in Figure 3, in combination with the preceding one, the step of playing 304 comprises a step of synchronizing 305, during the respective playing time, the video component V1 of the multimedia content CM1 and the audio component A1 of the multimedia content CM1 with each other.

The advantages of synchronizing were already described above with reference to the description of the system 200.

Moreover, if the user momentarily pauses the playing time of the video component V1 of the multimedia content CM1, for example by means of the user interface 101 of the exercise machine 100, the step of playing 304 may comprise a step of interrupting 305', by the portable electronic device 1 of the user, the playing time of the audio component A1 of the multimedia content CM1 up to a successive resumption of the playing time of the video component V1 of the multimedia content CM1.

Vice versa, if the user momentarily pauses the playing time of the audio component A1 of the multimedia content CM1, for example by means of the portable electronic device 1 of the user, the step of playing 304 comprises a step of interrupting 305", by the user interface 101 of the exercise machine 100, the playing time of the video component V1 of the multimedia content CM1 up to a successive resumption of the playing time of the audio component A1 of the multimedia content CM1.

Returning to Figure 3, in an embodiment, in combination with the preceding one, shown with dashed lines, the step of synchronizing 305 comprises a step of comparing 306 timestamps distributed along a playing time axis of the video component V1 of the multimedia content CM1 and timestamps distributed along a playing time axis of the audio component A1 of the multimedia content CM1, with each other.

According to a further embodiment, in combination with any one of those described above, shown with dashed lines in Figure 3, the step of providing 302 the video component V1 of the multimedia content CM1 comprises steps of:
- receiving 307, by the user interface 101 of the exercise machine 100, the video component V1 of the multimedia content CM1 from a remote electronic calculator 400 operatively connected to the user interface 101 of the exercise machine 100;
- playing 308, by the user interface 101 of the exercise machine 100, the video component V1 of the multimedia content CM1 received from the remote electronic calculator 400 operatively connected to the user interface 101 of the exercise machine 100.

In an embodiment, in combination with the preceding one, the step of streaming 303 the audio component A1 of the multimedia content CM1 is performed by the remote electronic calculator 400 operatively connected to the user interface 101 of the exercise machine 100.

According to an embodiment, alternatively to the preceding ones in which the video component V1 of the multimedia content CM1 is provided by the remote electronic calculator 400, shown with dashed lines in Figure 3, the step of providing 302 the video component V1 of the multimedia content CM1 comprises the steps of:
- storing 309 the multimedia content CM1 in a memory unit 103 of the user interface 101 of the exercise machine 100;
- playing 310, by the user interface 101 of the exercise machine 100, the video component V1 of the multimedia content CM1 stored in the memory unit 103 of the user interface 101 of the exercise machine 100.

In this embodiment, the step of streaming 303 the audio component A1 of the multimedia content CM1 stored in the memory unit 103 of the user interface 101 of the exercise machine 100 is performed by the user interface 101 of the exercise machine 100.

According to an embodiment, alternatively to the preceding ones, the step of providing 302 the video component V1 of the multimedia content CM1 comprises steps of:
- receiving 307, by the user interface 101 of the exercise machine 100, therefore the data processing unit 102, the video component V1 of the multimedia content CM1 from the remote electronic calculator 400 operatively connected to the user interface 101 of the exercise machine 100;
- playing 308, by the user interface 101 of the exercise machine 100, the video component V1 of the multimedia content CM1 received from the remote electronic calculator 400 operatively connected to the user interface 101 of the exercise machine 100.

In this embodiment, the step of streaming 303 the audio component A1 of the multimedia content CM1 is performed by the user interface 101 of the exercise machine 100.

In this embodiment, the audio component A1 of the multimedia content CM1 is stored in the memory unit 103 of the user interface 101 of the exercise machine 100 because the multimedia content CM1 was previously downloaded from the remote electronic calculator 400 (cloud).

In an embodiment, in combination with any one of those described above, the multimedia content CM1 is a video of a trainer preparatory to the performance of the workout.

The audio component A1 of the multimedia content comprises at least one first audio sub-component S1 representative of the voice of the trainer.

In this embodiment, shown with dashed lines in Figure 3, the method 300 comprises a step of:
- selecting 311, by the user, by means of the respective portable electronic device 1 or the user interface 101 of the exercise machine 100, as background music to be played together with the at least one first audio sub-component S1 representative of the voice of the trainer, a further audio component S3 representative of a background music.

The further audio component S3 representative of a background music is available in the portable electronic device 1 of the user.

According to a further embodiment, in combination with any one of the preceding ones, the audio component A1 of the multimedia content CM1 further comprises at least one second audio sub-component S2 representative of a background music.

The at least one second audio sub-component S2 is, for example provided by an audio streaming platform.

In this embodiment, shown with dashed lines in Figure 3, the method 300 comprises a step of:
- selecting 312, by the user, by means of the respective portable electronic device 1 or the user interface 101 of the exercise machine 100, as background music to be played together with the at least one first audio sub-component S1 representative of the voice of the trainer, one of the at least one second audio sub-component S2 representative of a background music of the audio component A1 of the multimedia content and the further audio component S3 representative of a background music, the further audio component S3 representative of a background music being available in the portable electronic device 1 of the user.

In an embodiment, in combination with any one of those described above, the further audio component S3 representative of a background music available in the portable electronic device 1 of the user is stored in the memory module 3 of the portable electronic device 1 of the user.

In an embodiment, alternatively to the preceding one, the further audio component S3 representative of a background music available in the portable electronic device 1 of the user is an audio which may be streamed on the portable electronic device 1 of the user (for example, Spotify).

The audio which may be streamed preferably is provided by the user to the portable electronic device 1 from a further remote electronic calculator 500 (diagrammatically shown in Figure 2), for example an audio streaming platform.

According to an embodiment, in combination with any one of those described above, wherein the step of playing 304 the audio component A1 of the multimedia content CM1 by means of an audio speaker 4 operatively associated with the portable electronic device 1 of the user is performed by means of a loudspeaker 4' of the portable electronic device 1 of the user.

According to a further embodiment, alternatively to or in combination with the preceding one, the step of playing 304 the audio component A1 of the multimedia content CM1 by means of an audio speaker A1 operatively associated with the portable electronic device 1 of the user is performed by means of a headset 4" operatively connected to the portable electronic device 1 of the user in a wireless or wired manner.

According to a further embodiment, in combination with any one of those described above and shown with dashed lines in Figure 3, the method 300 comprises a step of providing 313, by the user by means of the user interface 101 of the exercise machine 100, an activation command CT.

In this embodiment, the method 300 further comprises a step of receiving and recognizing 314, by the portable electronic device 1 of the user (therefore, by the respective data processing module 2) the activation command CT provided by the user by means of the user interface 101 of the exercise machine 100.

An example of activation command was provided above.

In this embodiment, the step of providing 302 the video component V1 of the multimedia content CM1, which may be used during the workout at the exercise machine 100 which may be employed by the user for the workout, and the step of streaming 303 the audio component A1 of the multimedia content CM1 to the portable electronic device 1 of the user adapted to perform the workout at the exercise machine 100, are performed following the establishment, by the portable electronic device 1 of the user, of the data connection with the user interface 101 of the exercise machine 100, which may be employed by the user during a workout in response to an activation command CT provided by the user by means of the user interface 101 of the exercise machine 100 and received and recognized by the portable electronic device 1 of the user.

According to a further embodiment, alternatively to or in combination with the preceding one and shown with dashed lines in Figure 3, the method 300 comprises a step of providing 315, by the user by means of the portable electronic device 1 of the user, an activation command CT.

In this embodiment, the method 300 further comprises a step of receiving and recognizing 316, by the user interface 101 of the exercise machine 100 (therefore, by the respective data processing unit 102), the activation command CT provided by the user by means of the portable electronic device 1 of the user.

An example of activation command was provided above.

According to this embodiment, the step of providing 302 the video component V1 of the multimedia content CM1, which may be used during the workout at the exercise machine 100 which may be employed by the user for the workout, and the step of streaming 303 the audio component A1 of the multimedia content CM1 to the portable electronic device 1 of the user, adapted to perform the workout at the exercise machine 100, are performed following the establishment, by the portable electronic device 1 of the user, of the data connection with the user interface 101 of the exercise machine 100 which may be employed by the user during a workout in response to an activation command CT provided by the user by means of the portable electronic device 1 of the user and received and recognized by the user interface 101 of the exercise machine 100.

The method 300 concludes with a symbolic step of ending ED.

With reference to an embodiment and the aforesaid drawings, an example is now described of the implementation, by the system 200, of a method for enjoying an audio component of a multimedia content during a workout.

A user gets on an exercise machine 100, for example, a treadmill like the one shown in Figure 1a, to perform a workout.

The user, by means of an activation command CT of the user interface 101 of the exercise machine 100, requests to use a multimedia content CM1, that is a video of a trainer preparatory to the performance of the workout he/she is starting on the exercise machine 100.

The multimedia content CM1 comprises an audio component A1 (voice of the trainer) and a video component V1 (video of the trainer).

Following the reception and recognition of the activation command CT, the portable electronic device 1 (smartphone) of the user establishes a data connection with the user interface 101 of the exercise machine 100.

The user interface 101 of the exercise machine 100 receives a video component V1 of a multimedia content CM1 from a remote electronic calculator 400 operatively connected to the exercise machine 100 and plays such a video component V1 of the multimedia content CM1 on a display module 104.

Moreover, the portable electronic device 1 of the user receives an audio component A1 of the multimedia content CM1 streamed by the remote electronic calculator 400 operatively connected to the user interface 101 of the exercise machine 100.

During the workout, the user uses the mutually synchronized video component V1 and audio component A1 of the multimedia content CM1 by means of the display module 104 of the user interface 101 of the exercise machine 100 and the audio speaker 4 operatively connected to the portable electronic device 1 of the user, for example a headset connected in a wireless manner (for example, in Bluetooth) to the smartphone, respectively.

If, in addition to the voice of the trainer (first audio sub-component S1), the audio component A1 also has a background music (second audio sub-component S2), the user can select the second audio sub-component S2 or a further audio component S3 available in the portable electronic device 1, that is the music which may be streamed on the smartphone (for example, Spotify), as background music by means of the portable electronic device 1.

Once the workout program is complete, use of the multimedia content CM1 also ends and the user gets off the exercise machine 100.

It is worth noting that the scope of the invention is fully achieved.

Indeed, the user is capable of enjoying the audio component A1 (voice of the trainer) directly on his/her portable electronic device 1 (smartphone) without any specific and complicated connection procedure of the headset or the speaker of the portable electronic device 1 of the user to the user interface of the exercise machine 100, therefore avoiding the need to ask for help or waste time with the subsequent slowing of the workout preparation operations.

Therefore, a timely enjoyment of the multimedia content CM1 is ensured within a workout class, which does not compromise the proper outcome of the workout with respect to the other timelier components of the workout class.

Moreover, the fact of receiving the streamed audio component A1 of the multimedia content allows quickly switching several exercise machines, if required within a set workout program.

Indeed, at each switching of exercise machine, when the portable electronic device 1 of the user is in the vicinity of the exercise machine 100, the data connection is established between the portable electronic device 1 of the user and the successive streaming, by the portable electronic device 1 of the user, of the audio component A1 of the multimedia content CM1.

The switching from one exercise machine to the successive one therefore occurs in the most natural manner possible, without distracting the user from the actual performance of the workout, thus making the experience perceived by the user during the workout as continuous, performing, satisfactory and pleasant as possible.

Moreover, the fact of being able to select between the background music S2 of the content CM1 and other background music S3 stored in the electronic device 1 of the user or which may be used by means of an audio streaming platform allows the user to personalize the content by selecting his/her favorite music.

A person skilled in the art may make changes and adaptations to the embodiments of the method and system described above or can replace elements with others which are functionally equivalent to satisfy contingent needs, without departing from the scope of protection of the following claims. Each of the features described as belonging to a possible embodiment may be implemented irrespective of the other embodiments described.

## Claims

1. A method (300) for enjoying an audio component (A1) of a multimedia content (CM1) during a workout, comprising steps of:
- establishing (301), by a portable electronic device (1) of the user, a data connection with a user interface (101) of an exercise machine (100) which may be employed by the user during a workout;
- providing (302) a video component (V1) of a multimedia content (CM1) which may be used during the workout at the exercise machine (100) which may be employed by the user for the workout;
- streaming (303) an audio component (A1) of the multimedia content (CM1) to the portable electronic device (1) of the user, adapted to perform the workout at the exercise machine (100);
- playing (304), by the portable electronic device (1) of the user, the audio component (A1) of the multimedia content (CM1) by means of an audio speaker (4) operatively associated with the portable electronic device (1) of the user during a playing time, by the user interface (101) of the exercise machine (100), of the video component (V1) of the multimedia content (CM1) by means of a display module (104) of the user interface (101) of the exercise machine (100).

2. A method (300) according to claim 1, wherein the step of playing (304) comprises a step of synchronizing (305), during the respective playing time, the video component (V1) of the multimedia content (CM1) and the audio component (A1) of the multimedia content (CM1) with each other.

3. A method (300) according to claim 2, wherein the step of synchronizing (305) comprises a step of comparing (306), with each other, timestamps distributed along a playing time axis of the video component (V1) of the multimedia content (CM1) and timestamps distributed along a playing time axis of the audio component (A1) of the multimedia content (CM1).

4. A method (300) according to any one of the preceding claims, wherein the step of providing (302) the video component (V1) of the multimedia content (CM1) comprises steps of:
- receiving (307), by the user interface (101) of the exercise machine (100), the video component (V1) of the multimedia content (CM1) from a remote electronic calculator (400) operatively connected to the user interface (101) of the exercise machine (100);
- playing (308), by the user interface (101) of the exercise machine (100), the video component (V1) of the multimedia content (CM1) received from the remote electronic calculator (400) operatively connected to user interface (101) of the exercise machine (100),
the step of streaming (303) the audio component (A1) of the multimedia content (CM1) being performed by the remote electronic calculator (400) operatively connected to the user interface (101) of the exercise machine (100).

5. A method (300) according to any one of the preceding claims 1 to 3, wherein the step of providing (302) the video component (V1) of the multimedia content (CM1) comprises steps of:
- storing (309) the multimedia content (CM1) in a memory unit (103) of the user interface (101) of the exercise machine (100);
- playing (310), by the user interface (101) of the exercise machine (100), the video component (V1) of the multimedia content (CM1) stored in the memory unit (103) of the user interface (101) of the exercise machine (100),
the step of streaming (303) the audio component (A1) of the multimedia content (CM1) stored in the memory unit (103) of the user interface (101) of the exercise machine (100) is performed by the user interface (101) of the exercise machine (100).

6. A method (300) according to any one of the preceding claims 1 to 3, wherein the step of providing (302) the video component (V1) of the multimedia content (CM1) comprises steps of:
- receiving (307), by the user interface (101) of the exercise machine (100), the video component (V1) of the multimedia content (CM1) from a remote electronic calculator (400) operatively connected to the user interface (101) of the exercise machine (100);
- playing (308), by the user interface (101) of the exercise machine (100), the video component (V1) of the multimedia content (CM1) received from the remote electronic calculator (400) operatively connected to the user interface (101) of the exercise machine (100),
the step of streaming (303) the audio component (A1) of the multimedia content (CM1) being performed from the user interface (101) of the exercise machine (100).

7. A method (300) according to any one of the preceding claims, wherein the multimedia content (CM1) is a video of a trainer, preparatory to the performance of the workout, the audio component (A1) of the multimedia content (CM1) comprising at least one first audio sub-component (S1) representative of the voice of the trainer, the method (300) comprising a step of:
- selecting (311), by the user, by means of the respective portable electronic device (1) or the user interface (101) of the exercise machine (100), as background music to be played together with the at least one first audio sub-component (S1) representative of the voice of the trainer, a further audio component (S3) representative of a background music, the further audio component (S3) representative of a background music being available in the portable electronic device (1) of the user.

8. A method (300) according to claim 7, wherein the audio component (A1) of the multimedia content (CM1) further comprises at least one second audio sub-component (S2), the method (300) comprising steps of:
- selecting (312), by the user, by means of the respective portable electronic device (1) or the user interface (101) of the exercise machine (100), as background music to be played together with the at least one first audio sub-component (S1) representative of the voice of the trainer, one of the at least one second audio sub-component (S2) representative of a background music of the audio component (A1) of the multimedia content and the further audio component (S3) representative of a background music, the further audio component (S3) representative of a background music being available in the portable electronic device (1) of the user.

9. A method (300) according to claim 8, wherein the further audio component (S3) representative of a background music available in the portable electronic device (1) of the user is stored in a memory module (3) of the portable electronic device (1) of the user or is an audio which may be streamed on the portable electronic device (1) of the user.

10. A method (300) according to any one of the preceding claims, wherein the step of playing (304) the audio component (A1) of the multimedia content (CM1) by means of an audio speaker (4) operatively associated with the portable electronic device (1) of the user is performed by means of a loudspeaker (4') of the portable electronic device (1) of the user and/or a headset (4") operatively connected in a wireless or wired manner to the portable electronic device (1) of the user.

11. A method (300) according to one of the preceding claims, wherein the step of providing (302) the video component (V1) of the multimedia content (CM1) which may be used during the workout at the exercise machine (100) which may be employed by the user for the workout, and the step of streaming (303) the audio component (A1) of the multimedia content (CM1) to the portable electronic device (1) of the user, adapted to perform the workout at the exercise machine (100), are performed following the establishment, by the portable electronic device (1) of the user, of the data connection with the user interface (101) of the exercise machine (100), which may be employed by the user during a workout in response to an activation command (CT) provided by the user by means of the user interface (101) of the exercise machine (100) and received and recognized by the portable electronic device (1) of the user.

12. A method according to any one of the preceding claims 1 to 10, wherein the step of providing (302) the video component (V1) of the multimedia content (CM1), which may be used during the workout at the exercise machine (100) which may be employed by the user for the workout, and the step of streaming (303) the audio component (A1) of the multimedia content (CM1) to the portable electronic device (1) of the user, adapted to perform the workout at the exercise machine (100), are performed following the establishment, by the portable electronic device (1) of the user, of the data connection with the user interface (101) of the exercise machine (100) which may be employed by the user during a workout in response to an activation command (CT), provided by the user by means of the portable electronic device (1) of the user and received and recognized by the user interface (101) of the exercise machine (100).

13. A system (200) for enjoying an audio component (A1) of a multimedia content (CM1) during a workout, comprising:
- an exercise machine (100) configured to receive a video component (V1) of a multimedia content (CM1) which may be used during the workout, the exercise machine (100) comprising a user interface (101) configured to play the video component (V1) of the multimedia content (CM1) by means of a display module (104) of the user interface (101) of the exercise machine (100);
- a portable electronic device (1) of the user configured to establish a data connection with the user interface (101) of the exercise machine (100) which may be employed by the user during a workout, the portable electronic device (1) of the user being configured to receive a streamed audio component (A1) of the multimedia content (CM1),
the portable electronic device (1) of the user being configured to play, by means of an audio speaker (4) operatively associated with the portable electronic device (1) of the user, the audio component (A1) of the multimedia content (CM1) during the playing time of the video component (V1) of the multimedia content by the display module (104) of the user interface (101) of the exercise machine (100).

14. A system (200) according to claim 13, comprising a remote electronic calculator (400) operatively connected to the user interface (101) of the exercise machine (100), the user interface (101) of the exercise machine (100) in order to provide the video component (V1) of the multimedia content (CM1) being configured to:
- receive the video component (V1) of the multimedia content (CM1) from the remote electronic calculator (400) operatively connected to user interface (101) of the exercise machine (100);
- play the video component (V1) of the multimedia content (CM1) received from the remote electronic calculator (400) operatively connected to the user interface (101) of the exercise machine (100),
the remote electronic calculator (400) operatively connected to the user interface (101) of the exercise machine (100) being configured to stream the audio component (A1) of the multimedia content (CM1).

15. A system (200) according to claim 13, wherein the user interface (101) of the exercise machine (100) comprises a memory unit (103) configured to store the multimedia content (CM1), the user interface (101) of the exercise machine (100) being configured to provide the video component (V1) of the multimedia content (CM1) by playing the video component (V1) of the multimedia content (CM1) stored in the memory unit (103) of the user interface (101) of the exercise machine (100),
the user interface (101) of the exercise machine (100) being configured to stream the audio component (A1) of the multimedia content (CM1) stored in the memory unit (103) of the user interface (101) of the exercise machine (100).

16. A system (200) according to claim 13, comprising a remote electronic calculator (400) operatively connected to the user interface (101) of the exercise machine (100), the user interface (101) of the exercise machine (100) being configured to:
- receive the video component (V1) of the multimedia content (CM1) from the remote electronic calculator (400) operatively connected to the user interface (101) of the exercise machine (100);
- play the video component (V1) of the multimedia content (CM1) received from the remote electronic calculator (400) operatively connected to the user interface (101) of the exercise machine (100);
- stream the audio component (A1) of the multimedia content (CM1).

17. A system (200) according to any one of the preceding claims 13 to 16, wherein the multimedia content (CM1) is a video of a trainer preparatory to the performance of the workout, the audio component (A1) of the multimedia content (CM1) comprising at least one first audio sub-component (S1) representative of the voice of the trainer, the portable electronic device (1) of the user or the user interface of the exercise machine (100) being configured to allow the user to select, as background music to be played together with the at least one first audio sub-component (S1) representative of the voice of the trainer, a further audio component (S3) representative of a background music available in the portable electronic device (1) of the user.

18. A system (200) according to any one of the preceding claims 13 to 16, wherein the audio component (A1) of the multimedia content (CM1) comprising at least one second audio sub-component (S2) representative of a background music, the portable electronic device (1) of the user or the user interface (101) of the exercise machine (100) being configured to allow the user to select, as background music to be played together with the at least one first audio sub-component (S1) representative of the voice of the trainer, one of the at least one second audio sub-component (S2) representative of a background music of the audio component of the multimedia content and a further audio component (S3) representative of a background music available in the portable electronic device (1) of the user.

19. A system (200) according to any one of the preceding claims 17 or 18, wherein the further audio component (S3) representative of a background music available in the portable electronic device (1) of the user is stored in a respective memory unit (3) of the portable electronic device (1) of the user or it is an audio which may be streamed on the portable electronic device (1) of the user.

20. A system (200) according to any one of the preceding claims 13 to 19, wherein the audio speaker (4) operatively associated with the portable electronic device comprises a loudspeaker (4') of the portable electronic device (1) of the user and/or a headset (4") configured to be operatively connected in a wireless or wired manner to the portable electronic device (1) of the user, the portable electronic device (1) of the user being configured to play the audio component (A1) of the multimedia content (CM1) by means of the loudspeaker (4') of the portable electronic device (1) of the user during the playing time of the video component (V1) of the multimedia content (CM1) by the user interface (101) of the exercise machine (100) by means of the display module (104) and/or by means of the headset (4") operatively connected in a wireless or wired manner to the portable electronic device (1) of the user during the playing time of the video component (V1) of the multimedia content (CM1) by the user interface (101) of the exercise machine (100) by means of the display module (104).

21. A system (200) according to any one of the preceding claims 13 to 20, wherein the user interface (101) comprises an activation command (CT), the portable electronic device (1) of the user being configured to receive and recognize the activation command (CT) provided by the user by means of the user interface (101) of the exercise machine (100), the user interface (101) of the exercise machine (100) and the portable electronic device (1) of the user adapted to perform the workout at the exercise machine (100) being configured, respectively, to receive the video component (V1) of the multimedia content (CM1) which may be used during the workout and to receive the streamed audio component (A1) of the multimedia content (CM1), following the establishment, by the portable electronic device (1) of the user, of the data connection with the user interface (101) of the exercise machine (100) which may be employed by the user during a workout, in response to the activation command (CT) provided by the user by means of the user interface (101) of the exercise machine (100).

22. A system (200) according to any one of the preceding claims 13 to 20, wherein the portable electronic device (1) of the user comprises an activation command (CT), the user interface (101) of the exercise machine (100) being configured to receive and recognize the activation command (CT) provided by the user by means of the portable electronic device (1), the user interface (101) of the exercise machine (100) and the portable electronic device (1) of the user adapted to perform the workout at the exercise machine (100) being configured, respectively, to receive the video component (V1) of the multimedia content (CM1) which may be used during the workout and to receive the streamed audio component (A1) of the multimedia content (CM1), following the establishment, by the portable electronic device (1) of the user, of the data connection with the user interface (101) of the exercise machine (100) which may be employed by the user during a workout, in response to the activation command (CT) provided by the user by means of the portable electronic device (1) of the user.
